# EUROPEAN PATENT APPLICATION

(11) **EP 1 538 220 A2**
(43) Date of publication of application: **08.06.2005**
(21) Application number: 03771120.7
(22) Date of filing: 23.07.2003
(51) Int. Cl.: C12Q 1/68

(54) **ALLELIC VARIANTS IN THE FACTOR VII GENE**

(30) Priority: 25.07.2002 ES 200201749
(71) Applicant: Fundacio privada i institut de recerca de l'hospital de la santa creu i sant pau, 08025 Barcelona (ES)
(72) Inventor: FONTCUBERTA BOJ, Jordi, 08025 Barcelona (ES); SORIA FERNANDEZ, Jose, Manuel, 08025 Barcelona (ES)
(74) Representative: Ponti Sales, Adelaida
(86) International application number: PCT/ES2003/000379
(87) International publication number: WO 2004/011675

(57) **Abstract**

The presence of at least one of said allelic variants affects the stability and/or functionality of the nucleic acid molecule and/or of the product coded by said nucleic acid molecule.

The procedure for analyzing a nucleic acid molecule comprises obtaining said molecule from a biological sample and determining at least one allelic variant from Table 1, said allelic variant affecting the stability and/or functionality of the nucleic acid molecule and/or of the product coded thereby.

The isolated product coded by a nucleic acid molecule which includes at least one allelic variant can be used as a medicament.

SEQUENCE LISTING

<110> Fundació privada e Institut de Recerca de l'Hospital de la
Santa Creu i Sant Pau.

<120> NEW ALLELIC VARIANTS IN THE FACTOR VII GENE

<130> A-157453

<140>
<141>

<150> ES 200201749
<151> 2002-07-25
<160> 36

<170> PatentIn Ver. 2.1

<210> 1
<211> 20
<212> ADN
<213> Artificial Sequence

<220>
<223> Description of Artificial Sequence: SEC.N°:1

<300>

<400> 1
atcccatata ttcttctgca                                              20

<210> 2
<211> 20
<212> ADN
<213> Artificial Sequence

<220>
<223> Description of Artificial Sequence:SEC.N°:2

<400> 2
agagcggacg gttttgttgc                                                    20

<210> 3
<211> 21
<212> ADN
<213> Artificial Sequence

<220>
<223> Description of Artificial Sequence: SEC. N°: 3

<400> 3
cggtcttgag atttgactcg c                                                  21

<210> 4
<211> 22
<212> ADN
<213> Artificial Sequence

<220>
<223> Description of the Artificial Sequence: SEC. N°: 4

<400> 4
cacacgatta tctggaagga ac                                                 22

<210> 5
<211> 19
<212> ADN
<213> Artificial Sequence

<220>
<223> Description of the Artificial Sequence: SEC. N°:5

<400> 5
cgcgggctga ggcaggttc                                                     19

<210> 6
<211> 19

<212> ADN
<213> Artificial Sequence

<220>
<223> Description of the Artificial Sequence: SEC. N°: 6

<400> 6
accacgtccc ttctgcgag                                                    19

<210> 7
<211> 21
<212> ADN
<213> Artificial Sequence

<220>
<223> Description of the Artificial Sequence: SEC.N°:7

<400> 7
tctagccgag acgtgctctt g                                                 21

<210> 8
<211> 20
<212> ADN
<213> Artificial Sequence

<220>
<223> Description of the Artificial Sequence: SEC.N°:8

<400> 8
cgagttgtca cgtcgtcctc                                                   20

<210> 9
<211> 20
<212> ADN
<213> Artificial Sequence

<220>
<223> Description of the Artificial Sequence: SEC.N°:9

<400> 9
actgtccccc ttgcaggagt                                                    20

<210> 10
<211> 19
<212> ADN
<213> Artificial Sequence

<220>
<223> Description of the Artificial Sequence: SEC. N°:10

<400> 10
ttctcattgg tcagcggct                                                     19

<210> 11
<211> 22
<212> ADN
<213> Artificial Sequence

<220>
<223> Description of the Artificial Sequence: SEC.N°:11

<400> 11
gggttcattt cagtgatgtt ga                                                 22

<210> 12
<211> 20
<212> ADN
<213> Artificial Sequence

<220>
<223> Description of the Artificial Sequence: SEC.N°:12

<400> 12
cggcacagcc aatgtctgta                                                    20

<210> 13

<211> 20
<212> ADN
<213> Artificial Sequence

<220>
<223> Description of the Artificial Sequence: SEC.N°:13

<400> 13
gccgttctcg ttcacacaga                                                    20

<210> 14
<211> 21
<212> ADN
<213> Artificial Sequence

<220>
<223> Description of the Artificial Sequence: SEC.N°:14

<400> 14
accttccagg cagaacacca c                                                  21

<210> 15
<211> 20
<212> ADN
<213> Artificial Sequence

<220>
<223> Description of the Artificial Sequence: SEC. N°:15

<400> 15
ccctgctttt ggaagtgcag                                                    20

<210> 16
<211> 20
<212> ADN
<213> Artificial Sequence

<220>

<223> Description of the Artificial Sequence: SEC.N°:16

<400> 16
gtgcctggtc agctgggtct                                                    20

<210> 17
<211> 21
<212> ADN
<213> Artificial Sequence

<220>
<223> Description of the Artificial Sequence: SEC.N°:17

<400> 17
gggctcaatg acatagaccc a                                                  21

<210> 18
<211> 20
<212> ADN
<213> Artificial Sequence

<220>
<223> Description of the Artificial Sequence: SEC.N°:18

<400> 18
gtgcgtgcat ccatgtgtat                                                    20

<210> 19
<211> 20
<212> ADN
<213> Artificial Sequence

<220>
<223> Description of the Artificial Sequence: SEC.N°:19

<400> 19
tttctaggtc tgcaggggct                                                    20

<210> 20
<211> 21
<212> ADN
<213> Artificial Sequence

<220>
<223> Description of the Artificial Sequence: SEC.N°:20

<400> 20
ccataaactt ggtggaaggg c                                                21

<210> 21
<211> 21
<212> ADN
<213> Artificial Sequence

<220>
<223> Description of the Artificial Sequence: SEC.N°:21

<400> 21
aggtctggag ctctcagggg t                                                21

<210> 22
<211> 20
<212> ADN
<213> Artificial Sequence

<220>
<223> Description of the Artificial Sequence: SEC.N°:22

<400> 22
tctccgcgtc cttgaagatc                                                  20

<210> 23
<211> 20
<212> ADN
<213> Artificial Sequence

<220>
<223> Description of the Artificial Sequence: SEC.N°:23

<400> 23
agcccctgca gacctagaaa                                                    20

<210> 24
<211> 20
<212> ADN
<213> Artificial Sequence

<220>
<223> Description of the Artificial Sequence: SEC.N°:24

<400> 24
agcacaggta ggggacggtg                                                    20

<210> 25
<211> 20
<212> ADN
<213> Artificial Sequence

<220>
<223> Description of the Artificial Sequence: SEC.N°:25

<400> 25
tgatcaacac catctgggtg                                                    20

<210> 26
<211> 20
<212> ADN
<213> Artificial Sequence

<220>
<223> Description of the Artificial Sequence: SEC.N°:26

<400> 26

tgggctcttg gtcaagtgag                                                    20

<210> 27
<211> 20
<212> ADN
<213> Artificial Sequence

<220>
<223> Description of the Artificial Sequence: SEC.N°:27

<400> 27
ggtgacgtgc acctgtggtc                                                    20

<210> 28
<211> 20
<212> ADN
<213> Artificial Sequence

<220>
<223> Description of the Artificial Sequence: SEC.N°:28

<400> 28
tggtcatctg ggtccagaat                                                    20

<210> 29
<211> 20
<212> ADN
<213> Artificial Sequence

<220>
<223> Description of the Artificial Sequence: SEC.N°:29

<400> 29
cctgaccatt gtctcctcag                                                    20

<210> 30
<211> 20
<212> ADN

<213> Artificial Sequence

<220>
<223> Description of the Artificial Sequence: SEC.N°:30

<400> 30
aagggacgtg gtgagaagct                                                    20

<210> 31
<211> 22
<212> ADN
<213> Artificial Sequence

<220>
<223> Description of the Artificial Sequence: SEC.N°:31

<400> 31
aaaaatgcta ggcatgacca tc                                                 22

<210> 32
<211> 22
<212> ADN
<213> Artificial Sequence

<220>
<223> Description of the Artificial Sequence: SEC.N°:32

<400> 32
cctcatgctc aaagaagcct ca                                                 22

<210> 33
<211> 21
<212> ADN
<213> Artificial Sequence

<220>
<223> Description of the Artificial Sequence: SEC.N°:33

<400> 33
cctgtcaaag acctcagact g                                                21

<210> 34
<211> 20
<212> ADN
<213> Artificial Sequence

<220>
<223> Description of the Artificial Sequence: SEC.N°:34

<400> 34
cccactttgg gtcccatatt                                                  20

<210> 35
<211> 22
<212> ADN
<213> Artificial Sequence

<220>
<223> Description of the Artificial Sequence: SEC.N°:35

<400> 35
tagagaagaa aatggctgct gc                                               22

<210> 36
<211> 20
<212> ADN
<213> Artificial Sequence

<220>
<223> Description of the Artificial Sequence: SEC.N°:36

<400> 36
tccagtctga ggtctttgac                                                  20

## Description

### FIELD OF THE INVENTION

The present invention relates to the field of cardiovascular diseases.
In particular, the present invention relates to the identification of new allelic variants in the factor VII gene sequence for determining the predisposition to a cardiovascular disease.

### BACKGROUND OF THE INVENTION

Factor VII is a vitamin K-dependent glycoprotein synthezised in the liver and secreted into the blood as an inactive zymogen at a concentration of 0.5 µg/ml² (Fair Blood, 1983). Following endothelial damage, the tissue factor (TF) is exposed and it binds to the Factor VII, setting up a coagulation reaction (Osterud. Proc. Natl. Acad. Sci. USA, 1977; Bauer et al., Blood, 1990).
The gene that encodes the Factor VII is located on 13q34-q.ter (Pfeiffer et al., 1982; Gilgenkrantz et al., 1986), contains 9 exons and 8 introns of 12.8 kb and codes for a protein of 406 amino acids. The complete gene sequence for human Factor VII was determined by O'Hara el al. (O'Hara P.J. et al., "Nucleotide sequence of the gene coding for human factor VII, a vitamin K-dependent protein participating in blood coagulation"; Proc. Natl. Acad. Sci. U.S.A. 84:5158-5162 (1987)). The mRNA is polyadenylated in multiple positions and has an efficient differential splicing. The mature protein has a molecular mass of approximately 50 KDa.
The activated form of the factor VII consists on one heavy chain and one light chain, both coded by the same gene, and linked by a disulphur bond between the cysteine 135 and the cysteine 262 (Hagen et al., 1986). It contains two EGF domains (epidermal growth factor domain), one Gla domain (γ-carboxyglutamic acid domain) and one trypsin-like catalytic domain (Hagen et. al., Natl. Acad. Sci. USA, 1986).
The heavy chain includes the catalytic part of the molecule and the heavy chain contains the Gla domain involved in the Ca²⁺ binding and the membrane binding, which are essential for factor VII activity.
The factor VII heavy-chain variants involve the direct interference in the activation process or the interruption of the catalytic mechanism, whereas most of the light-chain variants interrupt the interactions with Ca²⁺ or with membrane components which results in dysfunctional molecules (Zheng et al., Blood Coagul. Fibrinol, 1996).
Heriditary factor VII deficiency is an uncommon disorder showing autosomal recessive inheritance with high penetrance and variable expressivity (Kupfer et al., 1960; Triplet et al., 1985). It has an incidence of 1 per 500,000 in the general population (Wulf and Hermann. Hum. Mutation 15, 2000) and was recognized for the first time by Alexander et al., 1951. Some of the factor VII gene mutations have been identified and affect all domains of the protein, although about 50% of said mutations affect the protease domain (Wulff and Hermann, Hum. mutation, 2000), which indicates that the loss of proteasa function is the main cause of factor VII deficiency.
In general, the most common forms of disorder involve the presence of dysfunctional factor VII, which consists in low antigen levels in the plasma and a lengthening of prothrombin time due to defective activity of these molecules.
An absence of factor VII activity in plasma causes severe haemorrhage shortly after birth; indeed, there are studies in which mice deficient in FVII due to targeted disruption of the factor VII gene suffered fatal haemorrhage in the peri-partum period (McVey et al., Hum. mutation, 2000).
Moreover, about 30-40% of the variation of FVIIa levels in the general population can be explained by the existence of polymorphisms in the FVII gene (Bernardi et al., Blood 1996). These polymorphisms or allelic variants nevertheless show different allelic frequencies in different populations (Green et al., Arterioscler. Thromb., 1991; Bernardi, Marchetti, Pinotti. Arterioscler. Thromb. Vasc. Biol., 1996).
These allelic variants have been associated with varied risk of suffering from cardiovascular diseases, although the studies in which such an association has been described are contradictory and in no instance conclusive (Girelli et al., New England. J. Med., 2000; Iacoviello et al., N. Eng. J. Med., 1988). Furthermore, all the studies suffer from design errors and lack of statistical power.
The design and methodology used to date to approach the study of cardiovascular disease were based on investigating for presence of the risk factor in healthy individuals (controls) and disease sufferers (cases) who were unrelated to each other. Where the hypothetical risk factor was observed more frequently (in statistical terms) in the cases than in the controls it was concluded that the disease was associated with the factor under study. Strictly, however, a relationship of association does not necessary imply causation. This type of study, so-called Association or Case/Control Study, is entirely unsuitable for investigating genetic causes of complex illnesses, such as cardiovascular disease (Gambaro et al., Lancet 2000). Conventional epidemiological studies basically serve to identify environmental causes of illness (such as the smoking habit and lung cancer, oral contraceptives and venous thrombosis, or a vitamin-C-deficient diet and scurvy), but are highly ineffective when it comes to locating the genes involved. However, owing to the widespread use of PCR techniques in clinical laboratories, there are large numbers of Association Studies that relate genetic variants (polymorphisms) in certain candidate genes with all kinds of illnesses. Much confusion has been caused, because the results relating to a single polymorphism are often contradictory. Neither has the study of cardiovascular disease, for both venous and arterial types, remained free from this methodological perversion nor from the attendant chaotic collection of results (Girelli et al., New Eng. J. Med., 2000; Iacoviello et al., N. Eng. J. Med. 1998).

### DESCRIPTION OF THE INVENTION

The present invention relates to a molecule of nucleic acid comprising a sequence of the gene that codes for factor VII, characterized in that said molecule includes at least one allelic variant, said allelic variant affecting to the stability and/or functionality of said nucleic acid molecule, of the product obtained by transcription of said nucleic acid molecule and/or of the product coded by said nucleic acid molecule.

In the present invention, "nucleic acid molecule" is taken to mean a DNA sequence from the gene coding for factor VII protein. The length of said sequence is not an essential or restrictive aspect of this invention.

In the present invention, "allelic variant" is taken to mean a genetic variation in the DNA sequence that codes for factor VII protein, said genetic variation involving a pathology, loss or gain of stability and/or functionality. In particular, said allelic variant can be a deletion, an insertion or a substitution.

In a first aspect of the invention, therefore, new allelic variants are provided which have been identified in the gene that codes for factor VII protein.

Surprisingly, the inventors of the present invention have found that said allelic variants affect not only the functionality of the factor VII, but also the levels at which that protein is found. This is due to the fact that said variants can affect not only the nucleic acid molecule (DNA) but also the one transcribed from said molecule (RNA) and the protein. For example, if the allelic variant gives rise to an increase of the RNA stability, higher plasma levels of factor VII protein will be obtained; if the allelic variant affects an exon (that is, a protein-coding region) the functionality of the factor VII will be affected; if the allelic variant is on an intron (that is, on a non-coding region) DNA and/or RNA stability can be affected, altering the blood levels of FVII (increasing or decreasing said levels).

In this invention, "said variant affecting stability and/or functionality" is taken to mean an allelic variant that leads to an increase or a reduction of the stability, whether of DNA or RNA, and/or to increase in or loss of FVII function.

The human gene sequence that codes for FVII is known in the art (sequence published by O'Hara, P. J.; Grant, F. J.; Haldeman, B. A.; Grey, C. L.; Insley, M. Y.; Hagen, F. S.; Murray, M. J.: "Nucleotide sequence of the gene coding for human factor VII, a vitamin K-dependent protein participating in blood coagulation". *Proc. Net.* *Acad. Sci.* 84: 5158-5162, 1987. Access number in PubMed ID:3037537).

The allelic variants identified in the present invention are located on the promoter region of intron 1, intron 2, exon 3, intron 3, intron 5, exon 6, intron 7, intron 8, exon 9, in the 3'-UTR region (region 3' untranslatable to protein, but contains regulating sequences), as shown in the following table:

**Table 1:**

| allelic variants identified in the present invention. SNP (*Single Nucleotide Polymorphism*) variation of a base (nucleotide) in the DNA sequence. | | | |
|---|---|---|---|
| **Nucleotide O'Hara et al.** | **Allelic Variant** | **Position** | **Type** |
| -3216 | C/T | Promoter | SNP |
| -2987 | C/A | Promoter | SNP |
| -668 | A/C | Promoter | SNP |
| -628 | A/G | Promoter | SNP |
| -402 | G/A | Promoter | SNP |
| -401 | G/T | Promoter | SNP |
| -323 | Ins 0/10 | Promoter | Insertion |
| -122 | T/C | Promoter | SNP |
| 73 | G/A | Intron 1 | SNP |
| 260 | A/G | Intron 1 | SNP |
| 364 | G/A | Intron 1 | SNP |
| 698 | T/C | Intron 1 | SNP |
| 705 | G/A | Intron 1 | SNP |
| 710 | C/G | Intron 1 | SNP |
| 723 | IVS1 | Intron 1 | VNTR |
| 799 | T/C | Intron 1 | SNP |
| 806 | G/A | Intron 1 | SNP |
| 811 | C/G | Intron 1 | SNP |
| 833 | T/C | Intron 1 | SNP |
| 3.171 | G/A | Intron 2 | SNP |
| 3.294 | G/A | Intron 2 | SNP |
| 3.380 | C/T | Intron 2 | SNP |
| 3.423 | G/T | Intron 2 | SNP |
| 3.928 Q35Q | G/A | Exon 3 | SNP |
| 4.003 | G/A | Intron 3 | SNP |
| 5.191 | A/G | Intron 3 | SNP |
| 5.503 | T/A | Intron 3 | SNP |
| 6.331 | G/A | Intron 5 | SNP |
| 6.448 | G/T | Intron 5 | SNP |
| 6.452 | G/T | Intron 5 | SNP |
| 6.461 | IVS5 | Intron 5 | VNTR |
| 7.161 | G/C | Intron 5 | SNP |
| 7.453 | T/G | Intron 5 | SNP |
| 7.729 | G/A | Intron 5 | SNP |
| 7.880 H115H | C/T | Exon 6 | SNP |
| 8.695 | G/A | Intron 6 | SNP |
| 9.724 | IVS7 | Intron 8 | VNTR |
| 9.734 | A/G | Intron 8 | SNP |
| 9.779 | T/C | Intron 8 | SNP |
| 9.792 | G/A | Intron 8 | SNP |
| 9.847 | C/T | Intron 8 | SNP |
| 10.524 | G/A | Intron 8 | SNP |
| 10.534 | T/C | Intron 8 | SNP |
| 10.799 A294V | C/T | Exon 9 | SNP |
| 10.914 S333S | G/A | Exon 9 | SNP |
| 10976 R353Q | G/A | Exon 9 | SNP |
| 11.293 | Ins AA | 3'-UTR | Insertion |
| 11.622 | Del AG | 3'-UTR | SNP |
| 11.912 | G/A | 3'-UTR | SNP |

The numbering of the allelic variants described in the table is based on the numbering of the sequence of the human factor VII coding gene published by O'Hara.

The first column indicates the position in which an allelic variant was detected, taking O'Hara's published numbering sequence as a reference. The upper-case letters (in the second column) show which is the allelic variant in a specific position. For example, -3216 C/T means that in position -3216 (which is in the region of the promoter) the normal allele is a C (cytosine) and the allelic variant is T (thymidine); 11293 Ins AA means that two adenine nucleotides are inserted at position 11293; 11622 of the AG means that there is deletion of two nucleotides, adenine and guanine, at position 11622.

In a second aspect, the inventors of the present invention have found that identification of said allelic variants in a nucleic acid molecule indicates that the patient might develop a cardiovascular disease, due to the fact that said allelic variants may be functional (identified on the nucleic acid molecule's exons), affecting the total or partial function of the protein coded by said molecule and, therefore, having an effect on the coagulation process in which said protein is involved.

Indeed, the protein (factor VII) coded by a nucleic acid molecule in accordance with the present invention can have altered its stability, its secretion from the cell to the plasma, the average life in the plasma, etc.

The nucleic acid molecule can itself be altered by the presence of at least one of the allelic variants of Table 1, in terms of transcription rate, average life of messenger RNA, rate of translation to protein in the ribosomes, etc.

Due to all this, this invention relates to proteins coded by a nucleic acid molecule that includes at least one allelic variant of Table 1, for use as a medicament.

For example, if the allelic variant is located on an intron of said nucleic acid molecule, a more stable FVII protein that remains for a greater time in the plasma can be obtained and used for administration to patients with coagulation problems.

Another aspect of this invention is a procedure for analyzing a nucleic acid molecule, characterized in that it comprises obtaining said molecule from a biological sample and determining at least one allelic variant of Table 1, said allelic variable affecting the stability and/or functionality of said nucleic acid molecule and/or of the product coded thereby.

Under yet another aspect, this invention relates to a device for determining a predisposition to a cardiovascular disease which includes at least one of the oligonucleotides identified in Table 3 (see below).

Advantageously, if the patient's DNA sample is hybridized with at least one of the oligonucleotides identified in Table 3, this will indicate that it has at least one allelic variant in the factor VII gene and, therefore, the origin of the altered function of the factor VII can be determined.

A specific treatment can thus be designed for preventing a cardiovascular disease in patients who have not yet developed one but who present at least one allelic variant; a treatment can be designed that is specifically for palliating the factor VII dysfunction; or the product encoded by said nucleic acid molecule can be used to treat an illness associated with the coagulation cascade.

This invention therefore provides new allelic variants identified on the gene that codes for factor VII and affect the stability and/or functionality of said nucleic acid molecule and/or of the product coded thereby.

Advantageously, detection of said allelic variants not only permits detection of a predisposition to a cardiovascular disease (thrombosis-associated) but also means that the proteins that are coded by the nucleic acid molecules which include at least one of the allelic variants of Table 1 can be used as a medicament for the treatment of complications associated with thrombosis or with coagulation.

The following example is included by way of non-restrictive illustrative example.

### Examples

### Example 1: Procedure for identifying the allelic variants of the present invention.

### 1. Blood extraction

The DNA was extracted from white blood cells (leukocytes) from non-related persons with plasma FVII levels very much higher or lower than those which an expert in the art would consider to be normal for an average population. The blood samples were taken from the anticubital vein and anticoagulated immediately with 1/10 volume of sodium citrate 0.129 M.

The platelet-deficient plasma was obtained by centrifuging at 2000 g for 20 minutes, following which it was frozen and kept at -40°C until the time of analysis.

### 2. Isolation and amplification of the DNA.

The DNA was purified from the nuclei of leukocytes using the procedure described by Miller et al. (Miller et al., Nucl Ac Res 16 (3): 1215, 1988).

The FVII gene was analysed in various overlapping fragments that covered the entire gene sequence. The technique used for this analysis was Polymerase Chain Reaction (PCR). The primers used to amplify these fragments are shown in Tables 2 and 3.

**Table 2.**

| primers used to amplify the fragments obtained by PCR | | |
|---|---|---|
| | Amplification primers | Other internal primers |
| Fragment 1.1 | 71-72 | |
| Fragment 1.2 | 71.4-72 | |
| Fragment 2 | 73-74 | 73.1/73.2/74.1/74.3 |
| Fragment 3 | 75-76 | |
| Fragment 4 | 77-78 | 77.1/78.1 |
| Fragment 5.1 | 79B-710B | |
| Fragment 5.2 | 79C-710.1 | 710.1/710G |
| Fragment 6.1 | 711.2-712.1 | 711.5 |
| Fragment 6.2 | 711.1-712.2 | |
| Fragment 6.3 | 711.2-712 | 711.4 |
| Fragment 7 | 713-714 | 714.1/714.2/713.2/713.3 |
| Fragment 8 | 715-716 | 715.1/716.1 |

The various fragments of the FVII gene were numbered consecutively according to the order of analysis.

The primers were also numbered consecutively, the even numbers pertaining to forward primers and the odd numbers to reverse primers. Those skilled in the art know that in order to amplify a DNA fragment by PCR a forward primer and another reverse primer (complementing the DNA chain to be amplified) are always needed.

Included schematically are the sequences of the primers used (sequences NO:1 to NO:36).

**Table 3**

| Primer | SEQ. NO. |
|---|---|
| F715.1* | 1 |
| F72 | 2 |
| F73 | 3 |
| F74 | 4 |
| F77 | 5 |
| F78 | 6 |
| F712 | 7 |
| F713 | 8 |
| F714 | 9 |
| F715 | 10 |
| F716 | 11 |
| F711.2 | 12 |
| F712.1 | 13 |
| F711.1 | 14 |
| F712.2 | 15 |
| F710.1 | 16 |
| F711.3 | 17 |
| **Primer** | **SEQ. NO** |
| F716.1* | 18 |
| F714.1* | 19 |
| F73.1* | 20 |
| F77.1* | 21 |
| F78.1* | 22 |
| F713.2* | 23 |
| F73.2* | 24 |
| F713.3* | 25 |
| F714.2* | 26 |
| F71.4* | 27 |
| F711.5* | 28 |
| F711.4* | 29 |
| F79A | 30 |
| F710A | 31 |
| F79B | 32 |
| F710B | 33 |
| F79C | 34 |
| F710C | 35 |
| F710G | 36 |

The methodology followed for the PCR was standard in the art. Briefly, each fragment was amplified using GeneAmp PCR System 9700 (PE Applied Systems). The PCR products were generated in 50 µl of reaction mixtures containing 200 ng of genomic DNA, 0.5 U of Taq DNA polymerase (Biotaq DNA polymerase. Bioline), and the primers indicated in Tables 2 and 3, at a concentration of 0.5 µM each, the dNTPs at a concentration of 0.05 mM each, 1.5 mM MgCl₂ (1 mM MgCl₂ for fragment 1), and 5% DMSO (no DMSO in fragment 1 in the buffer for the PCR 1X Bioline).

The PCR programme was started with 5 min. at 94°C during the initial denaturation, followed by 30 cycles of amplification that consisted in 1 min. at 94°C, 1 min. at hybridisation temperature (57°C for fragments 1, 2, 7, 9 and 11, 59°C for fragments 3 and 8, and 61°C for fragment 10, of Table 1) and 2 min. at 72°C. The extension time was increased to 10 min. in the last cycle.

The amplified fragments were submitted to electrophoresis on an agarose gel at 1% for the control.

### 3. Sequencing of the DNA.

An enzymatic sequencing or dideoxynucleotides method was used, whose principle is based on the synthesis of a DNA chain by using a DNA polymerase based on a previously denatured DNA mould (fragment to be sequenced). In this case the PCR technique (mentioned above) was used, with four types of bases comprising the DNA in the form of dideoxynucleotides (ddNTPs), each marked with a different fluorescence.

Said fluorescence marker can be any of those known to those skilled in the art, such as the commercially available BigDyes (Apply-Biosystems).

It is at this step that synthesis of the DNA was interrupted by adding one of the ddNTPs. Thus a large number of fragments of different sizes were obtained and separated using continuous capillary electrophoresis. Each one of these fragments incorporates a fluorescent ddNTP that corresponds to a base making up the DNA chain. The colour of each fragment is determined when the fluorochrome (the ddNTP's fluorescent material) is excited by a laser, thereby producing a signal which is received by a photomultiplier and transmitted to a computer.

Computer analysis of the signals allows the sequence of the studied fragment to be determined. This technique is carried out using an automatic DNA sequencer (in our case, an ABI-310 model from Apply Biosystems).

All the allelic variants were identified by direct sequencing of the FVII gene.

The PCR-amplified fragments were purified, and the dNTPs and the unincorporated oligonucleotides removed using Quiagen QIAquick PCR Purification Kit columns before being sequenced.

The sequencing reaction was carried out in a volume of 10 µl, containing 3 µl of the purified DNA fragment, 4 µl of DNA Sequencing Kit BigDye Terminator Cycle Sequencing Ready Reaction (Applied Biosystems), 5% of dimethylsulfoxide (DMSO vol/vol) and 0.32 µM of the oligonucleotide for sequencing (Table 3).

The sequencing program comprised an initial step at 3' at 94°C, following by 25 cycles with the following routine: 10 seconds at 96°C, 5 seconds of hybridisation at 50°C and 4 minutes at 60°C. The sequences were carried out in an ABI PRISM 310 Genetic Analyzer.

Accordingly, the allelic variants of Table 1 were identified.

## Claims

1. Molecule of nucleic acid which comprises a sequence of the gene that codes for factor VII, **characterized in that** said molecule includes at least one allelic variant, said allelic variant affecting the stability and/or functionality of said nucleic acid molecule and/or of the product coded by said nucleic acid molecule.

2. Molecule of nucleic acid according to Claim 1, **characterised in that** the presence of at least one of said allelic variants is indicative of a predisposition to a cardiovascular disease.

3. Molecule of nucleic acid according to Claim 1 or 2, in which said allelic variant is one of those identified in Table 1.

4. Isolated product coded by a nucleic acid molecule according to any of Claims 1 to 3, for use as a medicament.

5. Allele-specific oligonucleotide which hybridizes with a nucleic acid molecule as claimed in any of Claims 1 to 3, in which the nucleotide of the polymorphic locus of said allele-specific oligonucleotide is different from the nucleotide of the polymorphic locus of the reference allele.

6. Oligonucleotide as claimed in Claim 5, **characterised in that** it is a probe.

7. Oligonucleotide as claimed in Claim 5, **characterised in that** it is one of those identified in Table 3.

8. Procedure for analysis of a nucleic acid molecule, **characterised in that** it comprises obtaining said molecule from biological sample and determining at least one allelic variant from Table 1, said allelic variant affecting the stability and/or functionality of the nucleic acid molecule and/or of the product coded thereby.

9. Diagnostic device for determining a predisposition to a cardiovascular disease, **characterised in that** it includes an oligonucleotide according to any of Claims 5 to 7.
